# EUROPEAN PATENT SPECIFICATION

(11) **EP 2 683 314 B1**
(45) Date of publication and mention of the grant of the patent: **31.05.2017**
(21) Application number: 11709260.1
(22) Date of filing: 08.03.2011
(51) Int. Cl.: A61B 17/86, A61B 17/17

(54) **FLEXIBLE HELICAL FIXATION DEVICE**
SPIRALFÖRMIGE FLEXIBLE FIXIERVORRICHTUNG
DISPOSITIF DE FIXATION HÉLICOÏDAL FLEXIBLE

(43) Date of publication of application: 15.01.2014
(73) Proprietor: Synthes GmbH, 4436 Oberdorf (CH)
(72) Inventor: SAIDHA, Sean, CH-4436 Oberdorf (CH); WHITE, Michael, CH-4436 Oberdorf (CH)
(74) Representative: Atkinson, Ian Anthony
(86) International application number: PCT/US2011/027478
(87) International publication number: WO 2012/121705

(56) References cited:
- EP-A1- 0 913 129
- WO-A1-2010/017631
- WO-A1-2010/111802
- DE-A1- 19 743 048
- DE-C- 908 906

## Description

Vertebral fixation (or spinal fixation) is a neurosurgical procedure in which two or more vertebrae are anchored to each other through a synthetic vertebral fixation device. The purpose of the vertebral fixation device is to reduce vertebral mobility in order to mitigate the risk of damage to the spinal cord or spinal nerve roots. A vertebral fixation procedure may be necessary to address instances of vertebral deformity, degenerative vertebral disorders (such as spondylolisthesis), or vertebral fractures.

Today spinal pathologies are being treated more and more often using minimally invasive posterior transpedicular or extrapedicular approaches to emplace spinal implant devices. The devices used to achieve vertebral fixation are often some type of permanent rigid or semi-rigid device made of titanium, titanium alloys, polyetheretherketone or carbon fibre amongst others and may comprise rods, plates, spacers, and various combinations thereof. In order to maintain a spinal implant in place, some form of fixation to bone can be used. In other cases, a vertebral fixation device may be used without the use of supplemental fixation (posterior/plate fixation) in which case, fixation is performed through the vertebral fixation device itself.

The most common means for fixing a device to the bone is to use some form of screw (or threaded shaft). Common types of screws used in medical procedures include pedicle screws, facet screws, and plate screws. Stable device fixation can also be achieved using blades, nails, hooks, and other such means, but few (if any) of these approaches can provide the same degree of purchase achievable using a screw-type fixation device.

One of the main drawbacks of screws, however, is that they are rigid and inflexible. Consequently, when a screw (or multiple screws) must be emplaced into a fixed body at an awkward angle with respect to the surgical opening necessary for implanting the spacer or other spinal device, a larger or more extensive opening is necessary to properly engage the screws. Unfortunately, the use of a larger or extended opening may not be consistent with the performance of a "minimally invasive" procedure.

WO-A-2011/111802 discloses a bone plate which has holes in it for receiving fixation screws. The screws have an externally threaded conical head and a shaft which is formed as a helically wound thin rod. The shaft is fastened to a tabbed ring which is received around the head. Each of the holes in the plate has a threaded conical portion, and a longitudinal portion which extends outwardly from the conical portion. The head of the screw is received in the conical portion and the shaft passes through the plate through the longitudinal portion of the hole.

The invention provides fixation apparatus as defined in claim 1.

Fixation apparatus provided by the invention comprises a flexible fixation device comprising a threaded head coupled to a bendable helical structure, and a guide device comprising a threaded fitting corresponding to the threaded head and, optionally, a helical grooved channel corresponding to the helical structure.

The flexible fixation device comprises a threaded head and a helical structure coupled to the threaded head, in which the helical structure is bendable upon the application of force.

The guide device for the flexible fixation device in which the guide device comprises an encasement manufactured from a material of greater rigidity than the flexible fixation device, and a shaped channel comprising an ingress and an egress for the flexible fixation device, said channel further comprising a bending feature for bending the flexible fixation device.

To facilitate an understanding of and for the purpose of illustrating the invention, features and implementations are disclosed in the accompanying drawings, it being understood, however, that the invention is not limited to the precise arrangements and instrumentalities shown, and in which similar reference characters denote similar elements throughout the several views, and in which:
FIG. 1A is a three-dimensional view of a flexible fixation device.
FIG. 1B is a cross-sectional view of the flexible fixation device of FIG. 1A.
FIG. 1C is a cross-sectional view of the flexible fixation device of FIGS. 1A and 1B in a semi-implanted angled configuration.
FIG. 2 is a three-dimensional view of a flexible fixation device similar to that shown in FIG. 1.
FIG. 3A is cross-sectional view of a guide device.
FIG. 3B is a three-dimensional view of the guide device of FIG. 3A that uses two flexible fixation devices.
FIG. 4 is a cross-sectional view of the flexible fixation device of FIGS. 1A, 1B, and 1C fully inserted and engaged in a guide device such as that shown in FIG. 3A.
FIG. 5 is an operational flow diagram representing a method for utilizing a guide device together with a flexible fixation device.

In order to allow for minimally invasive insertion of both a spinal device and the fixation device(s) necessary to hold the spinal device in place and/or to provide adequate stability to eliminate the need for supplemental fixation, a flexible fixation device as used in the invention can provide adequate purchase into bone at angles that are awkward for properly engaging inflexible and rigid screws. The fixation device is comprised of a head to effect engagement of the device and to act as an insertion-stop for the device, as well as a helical portion of a suitable cross-sectional geometry to allow for adequate purchase into bone. The helical portion provides fixation as the helical form engages the bone over a relatively large surface area relative to the size of the entry point. Optionally, the helical portion may be configured to resemble a cork screw, or the helical portion may be configured to resemble a coiled spring. Regardless of configuration, the device offers both flexibility (to make angled insertion achievable) while retaining enough helical-directional rigidity to achieve the desired implantation and fixation.

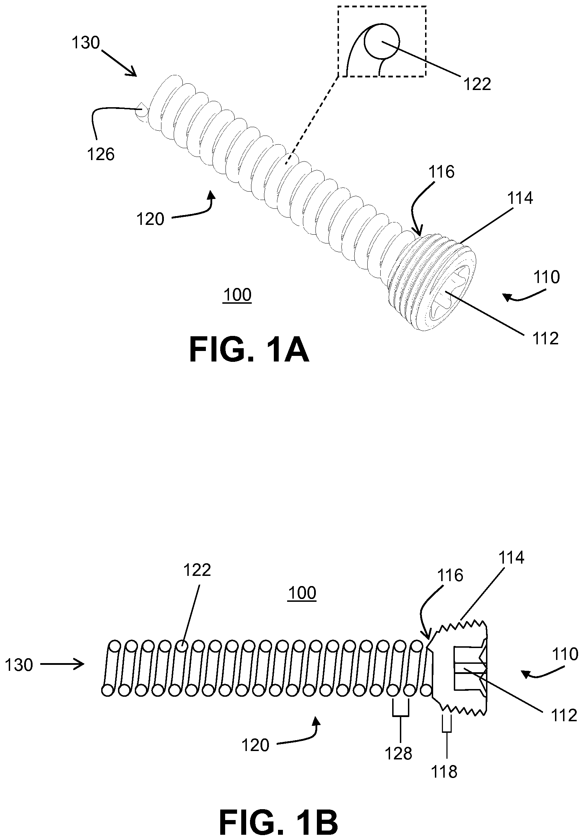

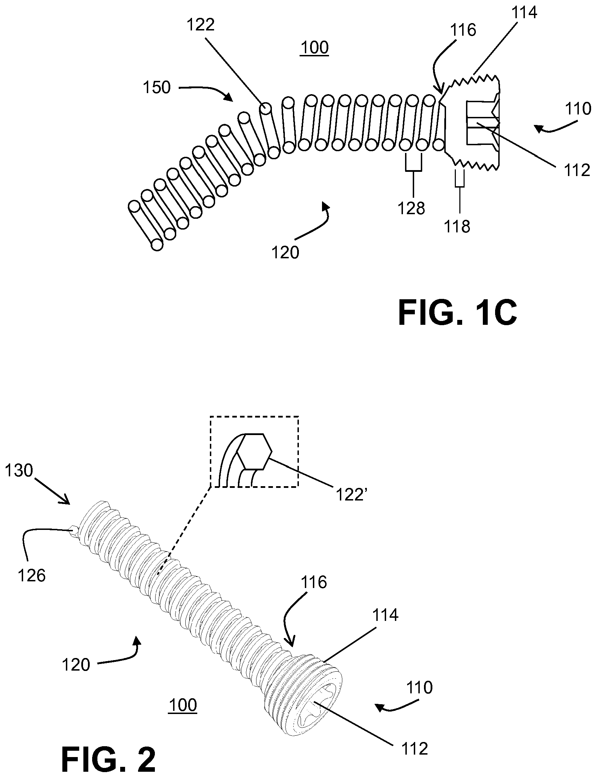

In FIGS. 1A, 1B, and 1C, the flexible fixation device 100 is formed to resemble a typical threaded shaft (or screw) and comprises a threaded head 110 featuring a turning tool engagement 112, exterior threads 114, and insertion-stop surface 116, as well as a helical structure 120 comprising a shaft having a suitable crossed-sectional geometry 122 (circular in this particular device) coiled around a hollow core 130 running the length of the helical structure 120. Located at the distal end of the helical portion furthest from the head is a sharpened tip 126 to provide efficient cortical bone penetration. The pitch 118 of the threaded head 110 may equal the pitch 128 of the helical portion 120.

The helical structure 120 achieves fixation as its coils gain adequate purchase into the bone over a large area compared to the size of the entry point. Optionally, the helical structure may resemble the screw-portion found on a corkscrew but having enough flexibility to be directionally redirectable (from a first or initial direction to a second or subsequent direction) during insertion (e.g., by a guide device having a stiffer composition) and yet is itself stiffer than the bone it is engaging in order to retain its coiled structure and provide the desired fixation. Optionally, the helical structure may be formed such that subsequent helical coils are spaced further apart than the cross-sectional diameter of the coils, or the helical structure may be formed such that the subsequent helical coils are spaced a distance equal to the cross-sectional diameter of the coils. In FIG. 1C, the semi-implanted angled configuration of the flexible fixation device 100 highlights the flexibility of the helical structure 120 at the bend point 150 that can occur anywhere along the length of the helical structure 120 and typical progresses from the tip 126 to a final position more proximal to the threaded head 110.

FIG. 2 is a three-dimensional view of a flexible fixation device similar to that shown in FIG. 1 but featuring a helical structure 120 comprising a shaft having hexagonal cross-sectional geometry 122'. Devices employing alternative cross-sectional geometries are also anticipated. For example, the cross-sectional geometries utilized by some alternative devices may include serrations, barbs, or edges to affix the device better in bone or to hinder the withdrawal of the device from bone once implanted.

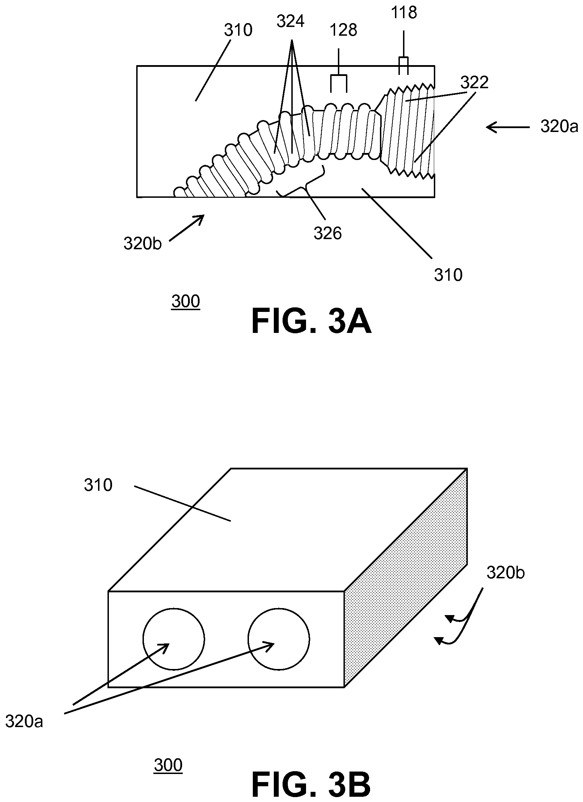

To effect the desired change in angle of the flexible fixation device 100, a guide device may be used. FIG. 3A is cross-sectional view of a guide device 300 which may be part of another device, for example, an interbody spacer for placement between adjacent vertebrae in the vertebral column. As shown in FIG. 3A, the guide device 300 comprises an encasement 310 manufactured from composition of stiffer and more inflexible qualities than the flexible fixation device 100. The encasement further comprises a shaped channel (extending from 320a to 320b) with an ingress 320a and an egress 320b for a flexible fixation device. The ingress 320a features a threaded fitting 322 matching the pitch 118 of the threaded head 110 of a flexible fixation device 100. Beyond the threaded fitting 322 lies a helical-grooved channel 324 matching the overall diameter of the helical structure 120 of a flexible fixation device 100 and shaped to match the pitch 128 conform to the individual coils comprising the helical structure 120.

The helical-grooved channel 324 further comprises a bending feature 326 that guides the coils of the helical structure 120 into a new direction corresponding to a desired resultant angle different from the direction of travel at the ingress 320a. The bending feature 326 initially distorts the spacing between the coils of the helical structure 120 to make the bend and change the direction of travel, and then the bending feature 326 returns the coils to the helical structure 120 to roughly their original spacing between the coils before such coils exit the encasement 310 at the egress 320b.

FIG. 3B is a three-dimensional view of the guide device of FIG. 3A that uses two flexible fixation devices. FIG. 3B shows that the encasement 310 of the guide device 300 may be adapted to receive plural fixation devices. Although two ingress 320a and egress 320b locations are shown, additional ingress 320a and egress locations 320b may be provided.

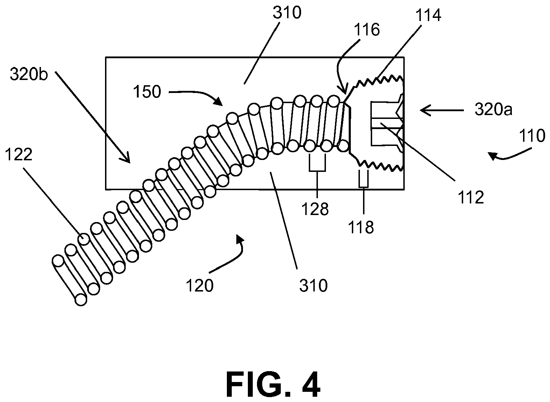

FIG. 4 is a cross-sectional view of the flexible fixation device 100 of FIGS. 1A, 1B, and 1C fully inserted and engaged in a guide device 300 such as that shown in FIG. 3A.

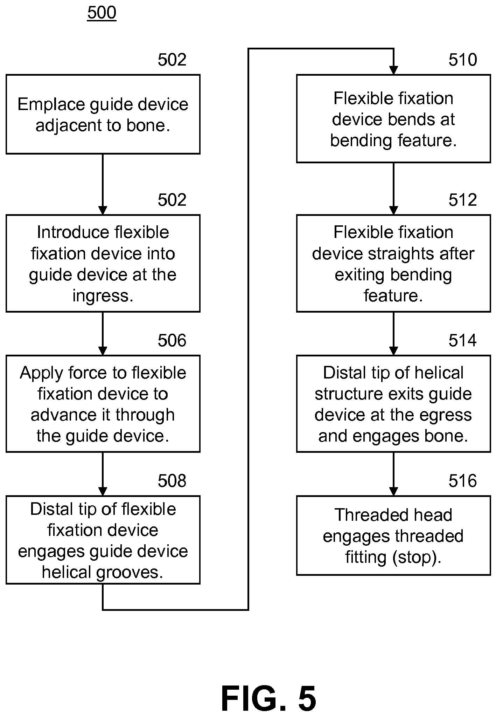

FIG. 5 is an operational flow diagram representing a method 500 for utilizing a guide device 300 together with a flexible fixation device 100. As shown in FIG. 5, at 502 the guide device 300 is emplaced adjacent to bone and ready to be fixed in place. At 504, the flexible fixation device 100 is introduced into the guide device 300 at the ingress 320a. At 506, force (such as a rotational force) is applied to the flexible fixation device 100 to cause it to turn within and advance through the guide device 300. At 508, the distal tip 126 of the fixation device engages a helical groove in the guide device 300. At 510 continued force applied to the flexible fixation device 100 causes the helical structure 120 to bend as it enters the bending feature 324. At 512 continued force applied to the flexible fixation device 100 causes the helical structure 120 to straighten again after passing through the bending feature 324. At 514, and with continued force applied to the flexible fixation device, the distal tip 126 of the helical structure 120 exits the egress and engages bone. At 516, upon further force the threaded head 110 engages into its threaded fitting 322 on the guide device 300.

Optionally, more than one flexible fixation devices 100 may be used to secure a particular guide device 300 in place. Optionally, a guide wire can be used, positioned through the central channel of the guide device 300 (between the ingress 320a and the egress 320b). The flexible fixation device may be used in a fixation target other than bone such as, for example, wood, plaster, rock, or any other solid materials. The guide device need not be inserted but can operate external to the fixation target.

## Claims

1. A fixation assembly which comprises
(a) a flexible fixation device (100) comprising a threaded head (110), and a helical structure (120) coupled to the threaded head, in which an application of a rotational force to the threaded head causes the helical structure to advance in a first direction, and in which the helical structure is bendable to advance in a second direction upon continued application of the rotational force, and
(b) a guide device (300) comprising an encasement (310) formed from a material of greater rigidity than the flexible fixation device (100), the guide device including a threaded fitting (322) corresponding to the threaded head, and a channel (324) having an ingress (320a) and an egress (320b) for the flexible fixation device,
**characterised in that** the channel (324) in the guide device (300) is shaped so that it has a bending feature (326) for bending the flexible fixation device as the flexible fixation device travels between the ingress and the egress.

2. The fixation assembly of Claim 1 in which the threaded head (110) further comprises a turning tool engagement (112), and an insertion stop surface (116).

3. The fixation assembly of Claim 1 in which the helical structure (120) comprises a distal sharpened tip (126).

4. The fixation assembly of Claim 1 in which the helical structure (120) comprises a shaft coiled around a hollow core (130).

5. The fixation assembly of Claim 1 in which the pitch of the threaded head (110) is equal to the pitch of the helical structure (120).

6. The fixation assembly of Claim 1 in which the helical structure (120) comprises a plurality of coils in which the space between a first coil and a second coil adjacent to the first coil is at least equal to a cross-sectional diameter of the first coil.

7. The fixation assembly of Claim 1 in which the helical structure (120) comprises a plurality of coils in which the space between a first coil and a second coil adjacent to the first coil is greater than a cross-sectional diameter of the first coil.

8. The fixation assembly of Claim 1 in which the helical structure (120) comprises a round cross-sectional geometry or a hexagonal cross-sectional geometry.

9. The fixation assembly of Claim 1 in which the helical structure (120) comprises a surface feature for affixing the flexible fixation device (100) in a bone or to hinder the withdrawal of the flexible fixation device from the bone once implanted.

10. The fixation assembly of Claim 9 in which the surface feature is one from among the following group of surface features: a serration, a barb, and an edge.

11. The fixation assembly of Claim 1 in which the shaped channel is a helically grooved channel (324) for engaging the helical structure (120) of the fixation device.

12. The fixation assembly of Claim 1 in which the bending feature (326), after bending the flexible fixation device (110), also unbends the flexible fixation device once a new direction of travel for the flexible fixation device has been achieved.

13. The fixation assembly of Claim 1 in which the pitch of the threaded head (110) is the same as the pitch of the helical structure (120), and in which both are the same as the pitch of the threaded fitting (322) and the pitch of the helical grooved channel (324).

14. The fixation assembly of Claim 1 in which the guide device (300) comprises an interbody spacer.

15. The fixation assembly of Claim 1 further comprising at least one additional flexible fixation device (100).

## Patentansprüche

1. Fixierungs- bzw. Befestigungsanordnung, die umfasst:
(a) eine flexible Fixierungs- bzw. Befestigungsvorrichtung (100), umfassend einen Gewindekopf (110) und eine wendelförmige bzw. spiralförmige Struktur (120), die mit dem Gewindekopf gekoppelt ist, wobei eine Aufbringung einer Drehkraft auf den Gewindekopf bewirkt, dass sich die wendelförmige bzw. spiralförmige Struktur in einer ersten Richtung vorwärtsbewegt, und wobei die wendelförmige bzw. spiralförmige Struktur biegbar ist, um sich bei anhaltender Aufbringung der Drehkraft in einer zweiten Richtung vorwärtszubewegen, und
(b) eine Führungsvorrichtung (300), umfassend eine Umhüllung (310), die aus einem Material größerer Steifigkeit gebildet ist als die flexible Fixierungs- bzw. Befestigungsvorrichtung (100), wobei die Führungsvorrichtung (300) ein Gewindepassstück (322) entsprechend dem Gewindekopf und einen Kanal (324) enthält, der einen Eingang (320a) und einen Ausgang (320b) für die flexible Fixierungs- bzw. Befestigungsvorrichtung aufweist,
**dadurch gekennzeichnet, dass** der Kanal (324) in der Führungsvorrichtung (300) so geformt ist, dass er ein Biegemerkmal (326) zum Biegen der flexiblen Fixierungs- bzw. Befestigungsvorrichtung aufweist, wenn sich die flexible Fixierungs- bzw. Befestigungsvorrichtung zwischen dem Eingang und dem Ausgang bewegt.

2. Fixierungsanordnung nach Anspruch 1, wobei der Gewindekopf (110) ferner einen Drehwerkzeugeingriff (112) und eine Einsetzstopfläche bzw. -oberfläche (116) umfasst.

3. Fixierungsanordnung nach Anspruch 1, wobei die spiralförmige Struktur (120) eine distale geschärfte Spitze (126) umfasst.

4. Fixierungsanordnung nach Anspruch 1, wobei die spiralförmige Struktur (120) einen Schaft umfasst, der um einen Hohlkern (130) herum gewickelt ist.

5. Fixierungsanordnung nach Anspruch 1, wobei die Steigung des Gewindekopfs (110) gleich der Steigung der spiralförmigen Struktur (120) ist.

6. Fixierungsanordnung nach Anspruch 1, wobei die spiralförmige Struktur (120) eine Mehrzahl von Wendeln bzw. Windungen umfasst, in denen der Raum zwischen einer ersten Wendel bzw. Windung und einer zweiten Wendel bzw. Windung angrenzend an bzw. benachbart zu der ersten Windung zumindest gleich einem Querschnittsdurchmesser der ersten Windung ist.

7. Fixierungsanordnung nach Anspruch 1, wobei die spiralförmige Struktur (120) eine Mehrzahl von Wendeln bzw. Windungen umfasst, in denen der Raum zwischen einer ersten Wendel bzw. Windung und einer zweiten Wendel bzw. Windung angrenzend an bzw. benachbart zu der ersten Windung größer als ein Querschnittsdurchmesser der ersten Windung ist.

8. Fixierungsanordnung nach Anspruch 1, wobei die spiralförmige Struktur (120) eine runde Querschnittsgeometrie oder eine hexagonal Querschnittsgeometrie umfasst.

9. Fixierungsanordnung nach Anspruch 1, wobei die spiralförmige Struktur (120) ein Flächen- bzw. Oberflächenmerkmal zum Fixieren bzw. Befestigen der flexiblen Fixierungsvorrichtung (100) in einem Knochen oder zum Verhindern des Herausziehens der flexiblen Fixierungsvorrichtung aus dem Knochen nach dem Implantieren.

10. Fixierungsanordnung nach Anspruch 9, wobei das Oberflächenmerkmal eines aus der folgenden Gruppe von Oberflächenmerkmalen ist: eine Einkerbung, ein Widerhaken und eine Kante.

11. Fixierungsanordnung nach Anspruch 1, wobei der geformte Kanal ein wendelförmig bzw. spiralförmig profilierter bzw. genuteter Kanal (324) zum Eingriff mit der spiralförmigen Struktur (120) der Fixierungsvorrichtung ist.

12. Fixierungsanordnung nach Anspruch 1, wobei das Biegemerkmal (326), nach dem Biegen der flexiblen Fixierungsvorrichtung (110), auch die flexible Fixierungsvorrichtung auseinander- bzw. geradebiegt, sobald eine neue Bewegungsrichtung für die flexible Fixierungsvorrichtung erreicht wurde.

13. Fixierungsanordnung nach Anspruch 1, wobei die Steigung des Gewindekopfs (110) die gleiche ist wie die Steigung der spiralförmigen Struktur (120), und wobei beide die gleichen sind wie die Steigung des Gewindepassstücks (322) und die Steigung des spiralförmig genuteten Kanals (324).

14. Fixierungsanordnung nach Anspruch 1, wobei die Führungsvorrichtung (300) einen Zwischenkörper- bzw. Wirbelkörperabstandshalter umfasst.

15. Fixierungsanordnung nach Anspruch 1, ferner umfassend zumindest eine zusätzliche flexible Fixierungs- bzw. Befestigungsvorrichtung (100).

## Revendications

1. Ensemble de fixation qui comprend :
(a) un dispositif de fixation flexible (100) comprenant une tête filetée (110) et une structure hélicoïdale (120) couplée à la tête filetée, dans lequel une application d'une force de rotation sur la tête filetée amène la structure hélicoïdale à avancer dans une première direction, et dans lequel la structure hélicoïdale peut être pliée pour avancer dans une seconde direction suite à l'application continue de la force de rotation, et
(b) un dispositif de guidage (300) comprenant une enveloppe (310) formée à partir d'un matériau ayant une rigidité supérieure au dispositif de fixation flexible (100), le dispositif de guidage comprenant un raccord fileté (322) correspondant à la tête filetée, et un canal (324) ayant une entrée (320a) et une sortie (320b) pour le dispositif de fixation flexible,
**caractérisé en ce que** le canal (324) dans le dispositif de guidage (300) est formé de sorte qu'il a une caractéristique de flexion (326) pour incliner le dispositif de fixation flexible lorsque le dispositif flexible se déplace entre l'entrée et la sortie.

2. Ensemble de fixation selon la revendication 1, dans lequel la tête filetée (110) comprend en outre une empreinte d'outil rotatif (112) et une surface d'arrêt d'insertion (116).

3. Ensemble de fixation selon la revendication 1, dans lequel la structure hélicoïdale (120) comprend une pointe pointue distale (126).

4. Ensemble de fixation selon la revendication 1, dans lequel la structure hélicoïdale (120) comprend un arbre enroulé autour d'un noyau creux (130).

5. Ensemble de fixation selon la revendication 1, dans lequel le pas de la tête filetée (110) est égal au pas de la structure hélicoïdale (120).

6. Ensemble de fixation selon la revendication 1, dans lequel la structure hélicoïdale (120) comprend une pluralité de spires, dans lequel l'espace entre une première spire et une seconde spire adjacente à la première spire est au moins égal à un diamètre transversal de la première spire.

7. Ensemble de fixation selon la revendication 1, dans lequel la structure hélicoïdale (120) comprend une pluralité de spires, dans lequel l'espace entre une première spire et une seconde spire adjacente à la première spire, est supérieur à un diamètre transversal de la première spire.

8. Ensemble de fixation selon la revendication 1, dans lequel la structure hélicoïdale (120) comprend une géométrie transversale arrondie ou une géométrie transversale hexagonale.

9. Ensemble de fixation selon la revendication 1, dans lequel la structure hélicoïdale (120) comprend une caractéristique de surface pour fixer le dispositif de fixation flexible (100) dans un os ou pour empêcher le retrait du dispositif de fixation flexible de l'os, une fois implanté.

10. Ensemble de fixation selon la revendication 9, dans lequel la caractéristique de surface est l'une parmi le groupe suivant de caractéristiques de surface : une dentelure, un ardillon et un bord.

11. Ensemble de fixation selon la revendication 1, dans lequel le canal formé est un canal à rainure hélicoïdale (324) pour mettre en prise la structure hélicoïdale (120) du dispositif de fixation.

12. Ensemble de fixation selon la revendication 1, dans lequel la caractéristique de pliage (326), après avoir incliné le dispositif de fixation flexible (110), redresse également le dispositif de fixation flexible une fois qu'une nouvelle direction de déplacement pour le dispositif de fixation flexible a été atteinte.

13. Ensemble de fixation selon la revendication 1, dans lequel le pas de la tête filetée (110) est le même que le pas de la structure hélicoïdale (120), et dans lequel tous deux sont les mêmes que le pas du raccord fileté (322) et le pas du canal à rainure hélicoïdale (324).

14. Ensemble de fixation selon la revendication 1, dans lequel le dispositif de guidage (300) comprend un dispositif d'espacement entre les corps.

15. Ensemble de fixation selon la revendication 1, comprenant en outre au moins un dispositif de fixation flexible (100) supplémentaire.
